# EUROPÄISCHE PATENTSCHRIFT

(11) **EP 0 349 795 B1**
(45) Veröffentlichungstag und Bekanntmachung des Hinweises auf die Patenterteilung: **08.12.1993**
(21) Anmeldenummer: 89110591.8
(22) Anmeldetag: 12.06.1989
(51) Int. Cl.: A61F 13/15

(54) **Wegwerfwindel**
Disposable diaper
Couche à jeter

(30) Priorität: 04.07.1988 DE 3822527
(43) Veröffentlichungstag der Anmeldung: 10.01.1990
(73) Patentinhaber: Paul Hartmann Aktiengesellschaft, D-89522 Heidenheim (DE)
(72) Erfinder: Malowaniec, Krzysztof, Dipl.-Ing., D-7920 Heidenheim (DE)
(74) Vertreter: Becker, Maria, Dipl.-Phys.

(56) Entgegenhaltungen:
- EP-A- 0 185 593
- GB-A- 2 011 791
- US-A- 2 627 858
- US-A- 3 881 488

## Beschreibung

Die Erfindung betrifft eine seitliche Beinschnitte aufweisende, längliche Wegwerfwindel nach dem Oberbegriff des Anspruchs 1.

Es sind bereits Einmalwindeln bekannt, die mit einer geschlechtsdifferenzierten Verstärkung des Saugkörpers versehen sind. Während die für Mädchen vorgesehene Windel eine symmetrische Saugkörperverstärkung im Schrittbereich aufweist, ist die für Knaben vorgesehene Windel im vorderen Bereich verstärkt. Derartige Vorschläge finden sich beispielsweise in dar EP-A- 0 202 125 und der EP-A- 0 185 593, sowie in der GB-A-2011791.

Bei diesen Windeln sind besondere Verstärkungszonen im vorderen Bereich und zwar von der Mitte bis zum Taillenbereich, angeordnet.

Diese zusätzlichen Verstärkungseinlagen haben den Nachteil, dass sie eine Inhomogenität des Saugkörpers bewirken, was sich beim Tragen unangenehm auswirkt. Auch wird die Flüssigkeitsverteilung durch die Grenzschichten zwischen dem Saugkörper und der Einlage gestört.

Neben dem herstellungstechnischen Aufwand hat die separate Anfertigung der Windeln für Knaben und Mädchen ferner den Nachteil, dass die differenzierten Windeln in unterschiedlichen Packungen untergebracht und angeboten werden müssen.

Der Erfindung liegt die Aufgabe zugrunde, eine Wegwerfwindel zu schaffen, die für beide Geschlechter gleich vorteilhaft verwendbar ist, indem deren Saugfähigkeit der bei beiden Geschlechtern unterschiedlichen Flüssigkeitsverteilung angepasst ist.

Diese Aufgabe wird durch die kennzeichnenden Merkmale des Anspruchs 1 gelöst. Bei der anatomischen Form der Wegwerfwindel mit asymmetrischem Beinausschnitt bewirkt dies, dass das Saugmaterial sowohl im mittleren Bereich als auch im vorderen Bereich stets gleiche Saugkapazität aufweist.

Wird aus herstellungstechnischen oder sonstigen Gründen eine Windel mit symmetrischen Beinausschnitten gewünscht, so kann gemäss einem weiteren Ausführungsbeispiel vorgesehen sein, des der Saugkörper integriert ist in einem zweiten aus saugfähigem Material bestehenden Trägerteil, der symmetrische Beinausschnitte aufweist.

In der Zeichnung sind Ausführungsbeispiele der Erfindung dargestellt. Es zeigen:
- Fig. 1: Eine Draufsicht auf einen ausgebreiteten Saugkörper einer Wegwerfwindel;
- Fig. 2: einen Längsschnitt des Saugkörpers in schematischer Darstellung sowie in ungepresstem Zustand;
- Fig. 3: eine Draufsicht auf einen weiteren modifizierten Saugkörper;
- Fig. 4: einen Längsschnitt des Saugkörpers gemäss Fig. 3 in schematischer Darstellung sowie in ungepresstem Zustand.

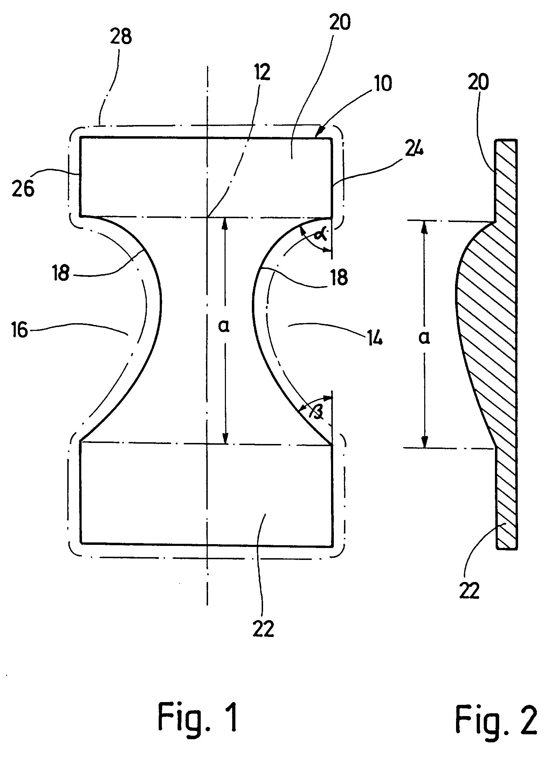

Der in Fig. 1 dargestellte Saugkörper 10 bildet einen im wesentlichen rechteckförmigen, länglichen Zuschnitt, der an beiden Längsseiten symmetrisch zu seiner Längssymmetrieachse 12 jeweils einen Beinausschnitt 14 bzw. 16 aufweist. Der Rand 18 dieser Beinausschnitte 14, 16 befindet sich somit zwischen den vorderen und hinteren Taillenbereichen 20 und 22 des Saugkörpers. Die Kontur der Beinausschnitte 14 und 16 bedingt, dass im vorderen Taillenbereich 20 der Rand 18 zusammen mit einer Verlängerung des Längsrandes 24 bzw. 26 des Saugkörpers einen Winkel α definiert, der gegenüber einem Winkel β im hinteren Taillenbereich 22 stumpfer ausgebiidet ist. Diese Gestaltung der asymmetrischen Beinausschnitte ermöglicht eine besonders körpergerechte Anpassung des Saugkörpers. In diesem Sinne ist auch der vordere Taillenbereich 20 zum hinteren Taillenbereich 22 kürzer ausgebildet.

Diese Konfiguration bringt es mit sich,dass üblicherweise sich im vorderen Taillenbereich 20 weniger saugfähiges Material befindet als im rückwärtigen Taillenbereich 22, sofern man einen Saugkörper von gleichmässiger Dicke und Dichte verwendet. In diesem Falle würde bei einem überwiegend im vorderen Taillenbereich 20 anfallenden Flüssigkeitsbereich, wie es bei Knaben der Fall ist, die Saugkapazität nicht ausreichen.

Um diesen Nachteil zu beheben, ist der Saugkörper 10 in dem durch die Beinausschnitte 14, 16 begrenzten Bereich a entlang der Längssymmetrieachse 12 an allen Stellen im wesentlichen mit der gleichen Menge an Saugmaterial ausgestattet.

Die asymmetrische Anordnung der Beinausschnitte 14, 16 zur Längsmitte des Saugkörpers 10 führt hierbei dazu, dass die Dicke bzw. Dichte im Schrittbereich in Richtung auf den vorderen Taillenbereich 20 grösser ist als in entgegengesetzter Richtung (Fig. 2). Dadurch wird erreicht, dass die Aufnahmekapazität im vorderen Taillenbereich 20 zumindest genauso gross ist wie im mittleren oder rückwärtigen Taillenbereich 22. Im fertiggestellten Zustand einer mit einem solchen Saugkörper 10 ausgestatten Windel weist der durch die Beinausschnitte 14, 16 begrenzte Bereich durch Flachpressen mittels einer Walze im wesentlichen die gleiche Dicke auf wie die vorderen und hinteren Taillenbereiche 20, 22.

Wie Fig. 1 zeigt, kann der Saugkörper im wesentlichen dem Zuschnitt einer Wegwerfwindel entsprechen, wobei er in diesem Falle zwischen einer flüssigkeitsundurchlässigen Wäscheschutzfolie sowie einer porösen Vliesstoffabdeckung eingehüllt ist. Mit 28 ist ein strichpunktiert dargestelltes Randstück der Wegwerfwindel bezeichnet, das aus den miteinander verbundenen Randteilen von Wäscheschutzfolie und Vliesstoffabdeckung gebildet ist.

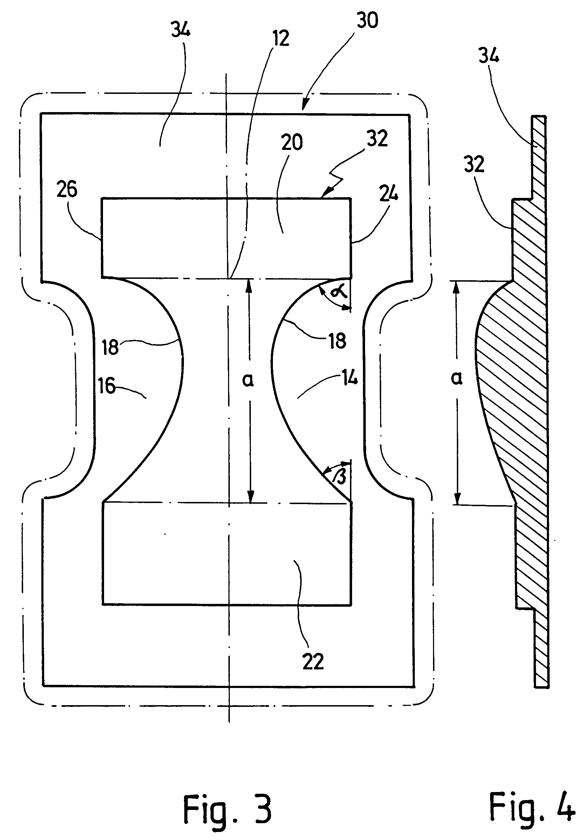

Beim Ausführungsbeispiel gemäss den Fig. 3 und 4 ist der vorstehend beschriebene Saugkörper 32 als Saugkörperkern in ein dieses aufnehmende Trägerteil 34 integriert, wobei beide Teile aus dem gleichen saugfähigen Material hergestellt sind. Der Saugkörperkern 32 entspricht in seiner Ausbildung und seinem Zuschnitt demjenigen des Saugkörpers 10 der Fig. 1 und 2, während das Trägerteil 34 im wesentlichen einem Windelzuschnitt mit symmetrischen Beinausschnitten entspricht und eine gleichmässige Dicke aufweist.

Die Herstellung des Saugkörpers 10 kann mittels vorgeformter Siebformen erfolgen, die eine entsprechende Matrix aufweisen.

## Patentansprüche

1. Seitliche Beinausschnitte aufweisende, längliche Wegwerfwindel, mit einer in angelegtem Zustand aussen liegenden, flüssigkeitsundurchlässigen Wäscheschutzfolie, einer innenseitig aufgebrachten, die Wäscheschutzfolie bedeckenden porösen Vliesstoffabdeckung und einem zwischen beiden angeordneten, sich vom Schrittbereich bis in die Taillenbereiche erstreckenden, dickeren Saugkörper, wobei der Rand der Beinausschnitte bogenförmig ausgebildet ist, wobei der Radius der Rundung der Beinausschnitte (14, 16) im Saugkörper im vorderen Bereich kleiner ist als in deren hinteren Bereich,
**dadurch gekennzeichnet,**
dass die Saugmaterialmenge des Saugkörpers (10) in dem durch die Beinausschnitte (14, 16) begrenzten Bereich (a) für jeden Flächenabschnitt entlang seiner Längssymmetrieachse (12) im wesentlichen konstant ist, wobei der jeweilige Flächenabschnitt gegeben ist durch das Produkt aus einem konstanten Längenabschnitt in Richtung der Längssymmetrieachse (12) und der jeweiligen Windelbreite.

2. Wegwerfwindel insbesondere nach Anspruch 1, dadurch gekennzeichnet, dass der Saugkörper (10) integriert ist in einem zweiten aus saugfähigem Material bestehenden Trägerteil (34), der symmetrische Beinausschnitte aufweist.

## Claims

1. Elongate disposable diapers having lateral cut-out sections for the legs, with a liquid-impermeable foil for protection of the underwear located on the outside in the worn state, a porous fleece cover attached on the inside and covering the foil for protection of the underwear, and a thicker absorbent body arranged between these two and extending from the stepping region to the waist regions, the edge of the cut-out sections for the legs being of arcuate design, and the radius of the curve of the cut-out sections (14, 16) for the legs in the absorbent body being smaller in the front region than in its rear region, characterized in that the amount of absorbent material of the absorbent body (10) in the region (a) delimited by the cut-out sections (14, 16) for the legs for each surface section along its longitudinal axis of symmetry (12) is essentially constant, the respective surface section being the product of a constant longitudinal section in the direction of the longitudinal axis of symmetry (12) and the respective diaper width.

2. Disposable diaper, in particular as defined in claim 1, characterized in that the absorbent body (10) is integrated in a second carrying part (34) consisting of absorbent material and having symmetrical cut-out sections for the legs.

## Revendications

1. Couche jetable de forme allongée, présentant des échancrures latérales pour les jambes, et une feuille protectrice de linge, imperméable aux liquides, située à l'extérieur lorsque la couche est en place, cette couche comportant un recouvrement en matière feutrée poreuse appliqué côté intérieur, recouvrant la feuille protectrice de linge, et comportant, agencé entre les deux, un corps absorbant plus épais s'étendant de la région de l'entrejambes jusque dans les zones de la taille, le bord des échancrures étant en forme d'arc, le rayon de l'arrondi des échancrures (14, 16) dans le corps absorbant étant plus petit dans la région avant que dans la région arrière,
caractérisée
par le fait que la quantité de matière absorbante du corps absorbant (10) dans la région (a) limitée par les échancrures (14, 16) pour les jambes est sensiblement constante pour chaque portion de surface le long de son axe longitudinal de symétrie (12), chaque portion de surface étant donnée par le produit d'une portion de longueur constante sur la direction de l'axe longitudinal de symétrie (12) par la largeur correspondante de la couche.

2. Couche jetable, notamment selon revendication 1, caractérisée par le fait que le corps absorbant (10) est intégré dans une deuxième partie support (34) qui est constituée de matière absorbante et présente des échancrures symétriques pour les jambes.
